# EUROPEAN PATENT APPLICATION

(11) **EP 4 464 252 A1**
(43) Date of publication of application: **20.11.2024**
(21) Application number: 23173371.8
(22) Date of filing: 15.05.2023
(51) Int. Cl.: A61B 6/03, A61B 6/00

(54) **A HOLDING DEVICE FOR HOLDING A MEDICAL IMAGING PHANTOM**

(71) Applicant: Positrigo AG, 8005 Zürich (CH)
(72) Inventor: AHNEN, Max Ludwig, 8005 Zürich (CH); FISCHER, Jannis Nikolaus Rudolf, 8049 Zürich (CH); KOPYLOV, Gleb, 8800 Thalwil (CH)
(74) Representative: Rutz, Andrea

(57) **Abstract**

A holding device (3) is provided of a medical imaging apparatus, in particular of a medical imaging apparatus in the form of a positron emission tomography (PET)-scanning apparatus (1). The holding device (3) serves for holding a medical imaging phantom (2) in a defined position inside a detector ring (11) of the medical imaging apparatus. The holding device (3) is adapted to be attached to an axial end face (113; 114) of the detector ring (111). Furthermore, a phantom unit comprising such a holding device (3) and a medical imaging phantom (2) is provided as well as a method for ensuring a quality standard of a medical imaging apparatus (1) and/or for calibrating a medical imaging apparatus (1).

## Description

### TECHNICAL FIELD

The present invention relates to a holding device of a medical imaging apparatus, such as a positron emission tomography (PET)-scanning apparatus, for holding a medical imaging phantom. Medical imaging phantoms are often used in medical imaging to ensure a quality standard and/or to calibrate the medical imaging apparatus.

### PRIOR ART

Medical imaging apparatuses, such as positron emission tomography (PET)-scanning apparatuses, magnetic resonance imaging (MRI) systems, computer tomography (CT)-devices and others, need to be calibrated on a regular basis, in order to ensure and maintain image quality. Also, the quality standard of the respective medical imaging apparatus needs to be verified from time to time, in order to guarantee a sufficient quality of the produced images, which are mainly used for diagnostic, but also for other purposes.

In order to ensure a quality standard and/or to calibrate the medical imaging apparatus, a medical imaging phantom with known specifications is usually placed inside of a detector ring of the medical imaging apparatus and is then scanned in accordance with a specific scanning setup and sequence provided for this purpose. Medical imaging phantoms are also often used for testing a medical imaging apparatus in research and development activities.

A medical imaging phantom comprising a hollow cylindrical case with a removable carrier that contains the radioactive sources is for example disclosed by US 4,499,375.

US 7,699,522 B2 discloses a quality assurance device in the form of a moving phantom for calibrating and testing the accuracy of movement correlated CT systems.

For positioning the medical imaging phantom at a desired position within the detector ring of the medical imaging apparatus, a holding device is usually provided. By means of the holding device, the medical imaging phantom is held at a defined position relative to the detector ring during e.g. the calibration- and/or quality-measurements. For this purpose, the holding device can be directly attached to the detector ring.

A direct attachment of a plurality of radioactive source loaders, which are used for calibrating a PET/CT-system, to the radial inner surface of a PET detector ring is for example disclosed by US 2008/0217541 A1. Each of the radioactive source loaders comprises a holding device for coupling a respective radioactive source pin to the inner surface of the detector ring.

A direct attachment of radioactive sources to the inner surface of the detector ring has certain advantages as concerns the precision, the accuracy and the reproducibility of the arrangement of the respective source in relation to the detector ring. On the other hand, the arrangement of the holding device serving to hold the radioactive source within the detector ring, i.e. within the normally imaged area, which is thus required, can have a detrimental effect on the measurement data. Also, the attachment of a radioactive source that serves as a medical imaging phantom to the radial inner surface of a detector ring can be inconvenient for the personnel, which in turn can lead to errors in the measurement, e.g. in the case of an incorrect arrangement of a medical imaging phantom.

JP 2008-11922 A discloses a medical imaging phantom that is attached to a moving table of a combined PET-/CT-system. The table, which is otherwise used to accommodate the patient to be scanned, can be moved into the bore of the detector ring with the medical imaging phantom attached to the table.

CN 215651191 U discloses a mounting block for clamping and holding a CT- or MRI-phantom. It seems that the mounting block is adapted to be positioned on a moving table of the scanner.

US 2016/0370445 A1 discloses another phantom, which is positioned on a moving table and is here used for co-registering magnetic resonance images with PET images.

WO 2017/158634 A1 discloses a medical imaging phantom for a CT-scanner, which is held by supports that are located in front and back positions with respect to the CT gantry.

In EP 3 646 793 A2, a phantom holder is disclosed for carrying a cylindrical phantom that is positioned along the longitudinal center axis of a CT-scanner. The phantom holder is attached to the supporting structure, which holds the detector ring.

The disadvantage of attaching a medical imaging phantom to the patient table or to another support structure separate from the detector ring is that the position and orientation of the phantom relative to the detector ring are generally not as precisely, accurately, and reproducibly defined as when attached directly to the detector ring. In particular with medical imaging apparatuses having a detector ring that can be displaced and/or rotated with respect to the structure to which the phantom is attached, there is an inherent risk of a certain deviation of the position and/or orientation of the phantom relative to the detector ring during measurement. However, depending on the phantom and/or the application, an exact and reproducible positioning of the medical imaging phantom within the detector ring of the medical imaging apparatus is often crucial, in order to guarantee the validity of the measured data.

### SUMMARY OF THE INVENTION

It is an object of the present invention to provide a holding device for holding a medical imaging phantom, which is easy in use and allows an exact and reproducible positioning of the phantom on a medical imaging apparatus.

This object is solved by a holding device of a medical imaging apparatus as claimed in claim 1. A phantom unit comprising such a holding device is claimed in claim 10. A method for ensuring a quality standard of a medical imaging apparatus and/or for calibrating a medical imaging apparatus is indicated in claim 13. Preferred embodiments are provided in the dependent claims.

The present invention thus provides a holding device of a medical imaging apparatus, in particular of a medical imaging apparatus in the form of a positron emission tomography (PET)-scanning apparatus, for holding a medical imaging phantom in a defined position inside a detector ring of the medical imaging apparatus.

The holding device is adapted to be attached to an axial end face of the detector ring.

By attaching the holding device to an axial end face of the detector ring, a precise, accurate and reproducible position and orientation of the phantom relative to the detector ring can be achieved, independently of a possible displacement and/or rotation of the detector ring relative to the supporting structure of the medical imaging apparatus. Furthermore, by attaching the holding device to an axial end face and not e.g. to the radial inner surface of the detector ring, the holding device or at least a large part thereof can be arranged outside of the imaged area, such that the influence of the holding device on the imaging result can be minimized. Also, an attachment of the holding device to an axial end face is usually considerably easier to accomplish by the personnel, than to a radial inner surface of the detector ring. Thus, with the holding device as indicated, a particularly easy and at the same time exact und reproducible positioning of the phantom on the medical imaging apparatus can be achieved.

The holding device thus serves to attach the medical imaging phantom to the medical imaging apparatus, in particular to the detector ring of the medical imaging apparatus. Preferably, the holding device is the only element that physically connects the phantom to the medical imaging apparatus, when the phantom is arranged inside the detector ring as intended, in order to e.g. perform a test or calibration scan. In a preferred embodiment, the holding device only connects the holding device to one axial end face of the detector ring. It would, however, also be conceivable in other embodiments that the holding device comprises a first and a second part, where the first part serves for attaching the phantom to a first axial end face and the second part serves for attaching the phantom to a second axial end face of the detector ring.

In use, the holding device is preferably arranged completely or to a large part outside of the detector ring and advantageously outside of the imaged area. In this way, the influence of the holding device on the imaging result can be minimized.

The holding device is preferably adapted to be releasably attached to the detector ring, advantageously in such a way, that no tools are needed for attaching and releasing the holding device to and from the detector ring. The procedure for doing e.g. the quality assurance and/or calibration scans can be considerably facilitated for the operator of the apparatus in this way. The holding device is preferably also adapted to be releasably attached to the medical imaging phantom, advantageously in such a way, that no tools are needed for attaching and releasing the holding device to and from the imaging phantom. This allows to store the phantom separately from the holding device and also to use the same holding device for different phantoms.

The medical imaging apparatus is preferably a positron emission tomography (PET)-scanning apparatus, but can also be a magnetic resonance imaging (MRI) system, a computer tomography (CT)-device or any other device adapted for medical imaging. In a particularly preferred embodiment, the medical imaging apparatus is particularly adapted for brain imaging, e.g. for obtaining images from the human or animal brain, with the imaged patient preferably being alive during the brain scanning procedure. Thus, the medical imaging apparatus can particularly be a brain PET-scanning apparatus. For this purpose, the detector ring of the medical imaging apparatus preferably has an inner diameter of 220 to 300 mm, more preferably of 240 to 280 mm, in particular of approximately 260 mm. Of course, a medical imaging apparatus, which is particularly adapted for a brain PET-scanning, can usually also be used for other purposes, such as e.g. the scanning of human extremities or for animal or plant scanning.

The detector ring of the medical imaging apparatus serves to obtain the imaging data during the scanning procedure. The part to be scanned of the patient is usually arranged radially within the detector ring during the scanning procedure. The detector ring is preferably closed along the circumferential direction. By forming a closed ring, the sensors or sensor modules for obtaining the imaging data can be optimally arranged along the entire inner surface of the detector ring. A closed ring also maximizes the detector sensitivity.

The detector ring preferably comprises a plurality of sensor modules which are usually all of identical design and which are usually all directed radially inwards. The sensor modules are advantageously arranged immediately next to each other along the circumference of the detector ring.

The inner surface is usually formed by a housing of the detector ring, which accommodates the sensor modules. Thus, the inner surface serves to separate an inner main opening of the detector ring and, thus, if it is a brain scanner, the patient's head, from the sensor modules.

Thus, the detector ring usually has a generally cylindrical housing with a radial inner and a radial outer surface as well as a first axial end face and a second axial end face. The holding device is preferably adapted to be attached to the first and/or the second axial end face of the housing. In a preferred embodiment, the holding device is adapted to be attached to one of the detector ring's axial end faces only. In alternative embodiments, the holding device can also be adapted to be additionally attached to the second end face and/or to the radial inner surface and/or to the radial outer surface of the detector ring.

While in most preferred embodiments the detector ring as a whole has a generally cylindrical shape, other embodiments are conceivable, in which the detector ring as a whole has a polygonal, such as e.g. a square or octagonal shape. In this case, both the radial inner and the radial outer surface of the detector ring preferably define a respective polygonal, in particular square or octagonal inner or outer circumference of the detector ring.

The medical imaging apparatus can have a main supporting structure to which the detector is attached. The detector ring can particularly be attached to the main supporting structure in such a way that it can be translationally displaced along e.g. a guide rail of the main supporting structure. Preferably, the detector ring can also be rotated and/or translationally displaced with respect to the main supporting structure of the medical imaging apparatus. The patient can then be in a sitting or lying position during the imaging procedure and the detector ring can be (fine-)positioned optimally with respect to the patient without having to move the patient. In normal use of the medical imaging apparatus, the main supporting structure usually carries the main weight, if not the entire weight, of the detector ring. Thus, the main supporting structure usually serves to support and to hold the detector ring in a stable position during the scanning procedure.

The holding device is preferably adapted to hold the medical imaging phantom in such a way, that the medical imaging phantom extends through the center of the detector ring and in particular along a main longitudinal axis of the detector ring. The center of the detector ring is usually the geometric center of the radial inner surface of the detector ring.

In a particularly preferred embodiment, the holding device comprises a plurality, in particular three, radially extending legs, which are each adapted to be attached to the axial end face of the detector ring. The legs preferably all extend in radial directions that are arranged within a common plane. Thus, the legs are preferably not inclined relative to each other. In this way, an overall flat configuration of the holding device can be achieved. An overall flat configuration of the holding device is not only particularly space saving, but also means that the holding device, when being attached to the detector ring as intended, usually does not extend into the inner space of the detector ring. The radially extending legs are preferably joined in a center portion of the holding device, which preferably coincides with the main longitudinal axis of the detector ring. The length of the radially extending legs from the center portion of the holding device to their free ends is preferably the same for each leg. The phantom is preferably attached or attachable to the holding device at the center portion of the holding device and advantageously in such a way that it extends in parallel or even coinciding with the main longitudinal axis of the detector ring.

The radially extending legs of the holding device can be arranged at regular angular distances relative to each other. Preferred, however, is an embodiment, in which the holding device as a whole has the shape of a Y, meaning that there are three radially extending legs and that the angular distance between a first and a second radially extending leg is smaller than the angular distance between a third radially extending leg and each of the first and the second leg. In this embodiment, the angular distance between the third radially extending leg and the first radially extending leg is preferably the same as the angular distance between the third radially extending leg and the second radially extending leg.

The holding device preferably comprises a coupling mechanism, in particular in the form of a latch mechanism, for releasably coupling the medical imaging phantom to the holding device. The coupling mechanism, which can particularly be a spring-loaded latch mechanism, is advantageously arranged in the center portion of the holding device. The provision of a coupling mechanism, in particular of a spring-loaded latch mechanism, allows a particularly easy attachment and removal of the imaging phantom to or from the holding device.

The coupling mechanism is preferably operatable by a user from the opposite side of the holding device with respect to the medical imaging phantom, when the medical imaging phantom is attached to the holding device. In this way, the imaging phantom can for example be removed easily from the holding device and inserted into a storage receptacle in a single step and without the user having to touch the imaging phantom. Also, the attachment of the imaging phantom to the holding device can be achieved in an analogous way, i.e. without the user having to touch the imaging phantom.

In a preferred embodiment, the holding device comprises a plurality, in particular three, attachment elements for attaching the holding device to the detector ring in an exact, pre-defined position. If the holding device comprises a plurality of radially extending legs, an attachment element is preferably arranged on each leg, advantageously in the region of the free end of each leg. The attachment elements thus serve to fixate the holding device on the axial face of the detector ring. For this purpose, respective complementary coupling elements are preferably provided on the axial face of the detector ring, of which each is assigned to one of the attachment elements of the holding device. By the positions of the attachment elements on the holding device and the positions of the coupling elements on the axial face of the detector ring, an exact and reproducible pre-defined positioning of the holding device on the detector ring can be achieved.

Preferably, each of the attachment elements forms a protrusion or indentation being adapted to engage a respective complementary indentation or protrusion provided on the axial face of the detector ring. The complementary indentations or protrusions on the detector ring in this case form the coupling elements. Due to the mutual engagement of a protrusion or indentation with a respective complementary indentation or protrusion, the pre-defined positioning of the holding device on the detector ring can be achieved in a particularly reproducible way.

For achieving a particularly easy attachment and removal of the holding device, each of the attachment elements can form a magnet or can be made of a ferromagnetic material, in order to be magnetically attracted by a respective ferromagnetic element or a magnet provided on the axial end face of the detector ring.

The present invention also relates to a phantom unit comprising the holding device as indicated above as well as a medical imaging phantom, which is adapted to be held by the holding device.

The medical imaging phantom or at least for the radioactive portion thereof preferably as a whole has the shape of a rod, i.e. has an overall length that is by an integer multiple larger than the diameter of the medical imaging phantom. Generally advantageous with regard to the quality assurance and the testing is a cylindrical overall shape of the medical imaging phantom or of the radioactive portion thereof. In certain embodiments, it can be advantageous to have a medical imaging phantom with an overall hollow cylindrical shape. In other alternative embodiments, it is also conceivable for the medical imaging phantom or at least for the radioactive portion thereof to have a spherical shape. The diameter of such a spherical medical imaging phantom or radioactive portion can have a diameter that is approximately as large or almost as large as the inner diameter of the detector ring, or it can have a diameter that is by an integer multiple smaller than the inner diameter of the detector ring. In the latter case, the medical imaging phantom can also be referred to as a "point source".

In order to facilitate the attachment of the phantom to the holding device, the holding device and the medical imaging phantom are preferably adapted to be attached to each other in an arbitrary rotational orientation relative to their respective main longitudinal direction. Thus, if a coupling mechanism is provided, the coupling mechanism is advantageously rotationally symmetric with respect to the main longitudinal axis of the imaging phantom and/or of the holding device.

According to the preferred embodiment, the phantom unit is adapted to ensure a quality standard of a PET-scanning apparatus and/or to calibrate a PET-scanning apparatus. For this purpose, the medical imaging phantom preferably comprises, an elongated cylinder source formed of a radioactive material, the radioactive material being advantageously made from the radionuclide ²²Na or ⁶⁸Ge.

Furthermore, the present invention relates to a method for ensuring a quality standard of a medical imaging apparatus and/or for calibrating a medical imaging apparatus, comprising the step of attaching a holding device, which is preferably a holding device as indicated above, to an axial end face of a detector ring of the medical imaging apparatus in such a way, that a medical imaging phantom is held in a defined position, in particular in a pre-defined position, inside the detector ring by the holding device.

By arranging the medical imaging phantom in a defined position relative to the detector ring, the obtained imaging data can be used for verifying the status and quality of the medical imaging apparatus and/or for calibrating the apparatus.

The attachment of the holding device with the medical imaging phantom to the axial end face of the detector ring is particularly easy for the personnel, because the end face, rather than the inner surface, is usually easily accessible. This not only makes positioning the phantom easy, but also less prone to error.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic side view of an exemplary medical imaging apparatus in the form of a brain PET-scanning apparatus which is adapted to be calibrated and/or to be tested by means of a medical imaging phantom held by an inventive holding device;
- Fig. 2: shows a perspective view of a medical imaging phantom which is adapted to be held by an inventive holding device;
- Fig. 3: shows a perspective view from above of an inventive holding device to which the attached medical imaging phantom of Fig. 2 is attached;
- Fig. 4a: shows a cross-sectional view of the holding device of Fig. 3 with the attached medical imaging phantom;
- Fig. 4b: shows an enlarged partial detail of the rectangular area framed by a dashed line in Fig. 4a;
- Fig. 4c: shows an enlarged partial detail of the rectangular area framed by a dash-dotted line in Fig. 4a;
- Fig. 5: shows a perspective view of the holding device of Fig. 3 from below, without the medical imaging phantom;
- Fig. 6: shows a perspective view from a first viewing direction of the holding device with the medical imaging phantom of Fig. 3 attached to the detector ring of a medical imaging apparatus;
- Fig. 7: shows a perspective view from a second viewing direction of the holding device with the medical imaging phantom of Fig. 3 attached to the detector ring of a medical imaging apparatus;
- Fig. 8a: shows a perspective view from a third viewing direction of the holding device with the medical imaging phantom of Fig. 3 attached to the detector ring of a medical imaging apparatus shown cut open;
- Fig. 8b: shows an enlarged partial detail of the rectangular area framed by a dashed line in Fig. 8a;
- Fig. 9: shows a perspective view of the medical imaging phantom of Fig. 2 stored in a storage container;
- Fig. 10: shows a perspective view of an attachment element of the holding device of Fig. 3;
- Fig. 11: shows a perspective view of a latch slider of the coupling mechanism of the holding device of Fig. 3; and
- Fig. 12: shows a perspective view of a coupling element of the detector ring of Fig. 6.

### DESCRIPTION OF PREFERRED EMBODIMENTS

Elements shown in the figures, which have the same or a similar function, but belong to different embodiments, are annotated with the same reference numerals in each case.

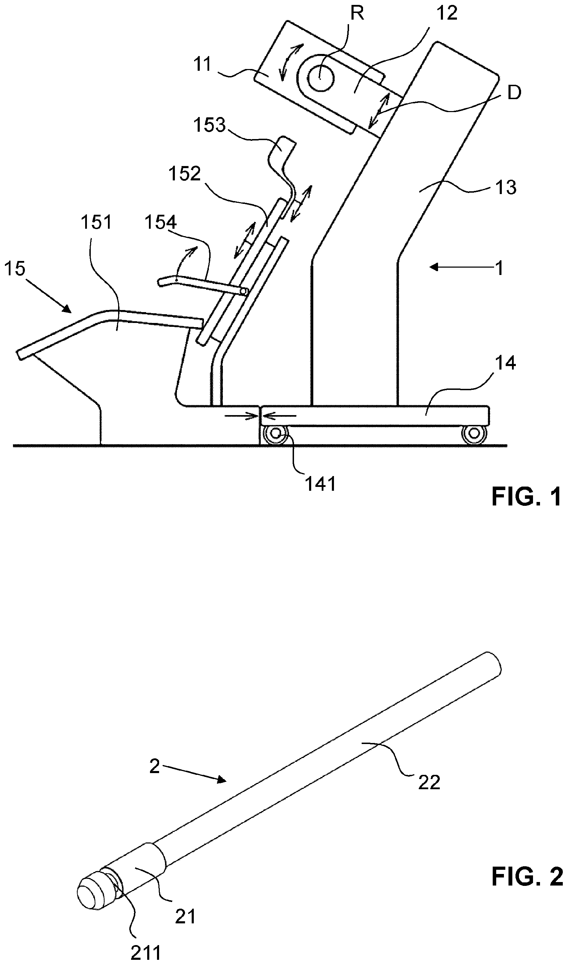

Figure 1 shows a medical imaging apparatus in the form of a PET-scanning apparatus 1 which is adapted to be calibrated and/or to be tested by means of a medical imaging phantom held by an inventive holding device. The embodiment of a PET-scanning apparatus 1 as shown in figure 1 is a brain-scanner, i.e. a scanner which is specifically adapted for the PET-imaging of the brain of human patients.

In the embodiment as shown in figure 1, the PET-scanning apparatus 1 comprises a detector ring 11 having a plurality of sensors or sensor modules arranged within its housing. The sensors or sensor modules, which each comprise a plurality of radially inwardly directed detector crystals, are not visible in the figures. They serve to detect and measure the PET-radiation emitted in the region of an inner main opening of the ring-shaped detector ring 11.

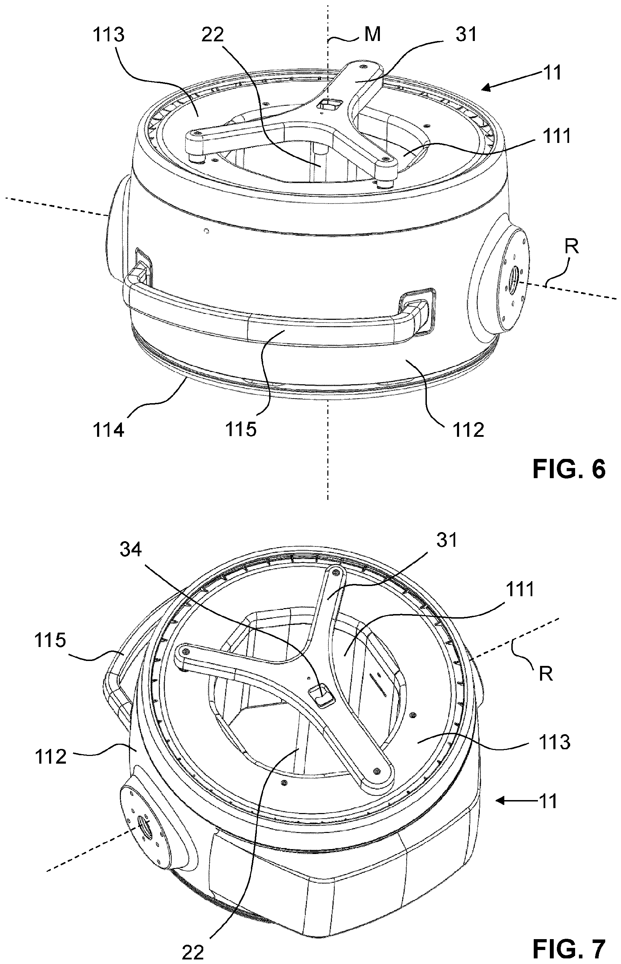

As can be seen from Figures 6 and 7, the detector ring 11 has an overall cylindrical shape with a radial outer surface 112 and a radial inner surface 111. The radial inner surface 111 circumferentially limits the inner main opening of the detector ring 11. The radial outer surface 112 and the radial inner surface 111 are connected at each end of the cylindrical shape of the detector ring 11 by a respective axial end face 113 and 114. Due to the cylindrical shape of the detector ring 11 and in particular of its radial inner surface 111, a main longitudinal axis M is defined as it is indicated by a dotted-dashed in Figure 6.

The detector ring 11 is arranged between two holding arms that hold the detector ring 11 between them. The two holding arms form a part of a U-shaped portion 12, which is attached to a main supporting structure 13. The detector ring 11 is rotatable about an axis of rotation R that extends diametrically through the detector ring 11 and through the two holding arms of the U-shaped portion 12 (see the respective dashed line in Figure 6). Furthermore, the detector ring 11 is displaceable together with the U-shaped portion 12 along an inclined displacement direction D relative to the main supporting structure 3. In order to facilitate rotation and displacement of the detector ring 11, one or several handles 115 can be attached to an outer surface of the detector ring 11. It is, however, also possible that the detector ring 11 is rotatable and/or displaceable by means of a respective motor which can be operated by an operator via e.g. respective buttons or via a touchscreen.

The direction along which the U-shaped portion 12 with the holding arms is displaceable is inclined with respect to the direction of gravity, meaning that it is neither parallel nor perpendicular to the direction of gravity. The U-shaped portion 12 is attached to a guide rail that is provided on the main supporting structure 13 and allows the U-shaped portion 12 to be displaced along the inclined displacement direction D as mentioned. Thus, the guide rail also extends along the inclined direction.

The main supporting structure 13 is mounted on a base structure 14, to which wheels 141 are attached that allow the part of the PET-scanning apparatus 1 with the detector ring 11 and the main supporting structure 13 to be moved easily.

A seating support 15 is provided for accommodating a human patient in an inclined sitting position during the scanning procedure. Prior to the image acquisition, the patient is accommodated on the seating support 15 with the detector ring 11 being in the uppermost position of the main supporting structure 3. The detector ring 11 is then displaced and, if necessary, rotated by the operator into an optimal position for the image acquisition.

The seating support 15 comprises a seat base 151 and an inclined backrest 152 for supporting the patient during the scanning procedure. Attached to the backrest 152 are pivotable armrests 154 as well as a headrest 153. For the image acquisition, the head of the patient rests on the headrest 153 and the detector ring 11 is displaced and rotated such that the patient's head is arranged inside of the detector ring 11. Thus, the headrest 153 is then likewise arranged inside of the detector ring 11 during the imaging acquisition process.

The displacement direction D of the detector ring 11, which is defined by the longitudinal extension of the respective guide rails, approximately corresponds to the longitudinal extensions of the backrest 152 and of the headrest 153. Thus, the displacement direction D of the detector ring 11 corresponds to the longitudinal main axis of the upper part of the body of the patient, if the patient sits on the seating support 15 as intended and is ready for the scanning procedure. In order to prevent the patient from moving his head during the image acquisition, a head strap can be used.

For assuring the quality and/or calibrating the PET-scanning apparatus, phantom scans are carried out on a regular basis. Daily routine tests can be performed by medical technologists, usually in the beginning of the morning and are often mandatory to ensure safety and a constant performance of the PET-scanning apparatus 1. The purpose of daily routine tests is to measure deviations from optimal performance of the PET-scanning apparatus 1 to indicate when a recalibration would be needed or to indicate when servicing would be needed. Deviations from optimal performance can particularly relate to image quality, sensitivity, and the general imaging performance of the PET-scanning apparatus 1. Thus, daily routine tests can particularly serve to detect specific problems of the PET-scanning device 1.

For carrying out the daily routine test, a medical imaging phantom 2 is used as it is shown in Figure 2. The medical imaging phantom 2, which as a whole has the shape of a rod, comprises an elongated radioactive cylinder portion 22 forming a radioactive source. Thus, the medical imaging phantom 2 has an overall length which is by an integer multiple larger than its diameter. The radioactive material contained in the radioactive cylinder portion 22 is preferably the radionuclide ²²Na or ⁶⁸Ge. In order to obtain a reproducible scan result from the measuring of the medical imaging phantom 2 in the daily routine test, the medical imaging phantom 2, which comprises a pre-known and well-defined distribution of the radioactive material within the radioactive cylinder portion 22, needs to be placed in a defined position inside the detector ring 11. In the present case, the medical imaging phantom 2 forms a line-source, meaning that the radioactive material is concentrated within a long, thin cylinder formed by the radioactive cylinder portion 22. For carrying out a daily routine test, the radioactive cylinder portion 22 is placed centrally and such within the detector ring 11, that the longitudinal main axis of the radioactive cylinder portion 22 coincides with the main longitudinal axis M of the detector ring 11.

In order to be placed and held within the detector ring 11, the medical imaging phantom 2 comprises a mounting portion 21 with a latch structure 211, that serves to releasably attach the medical imaging phantom to a holding device 3. The latch structure 211 is here formed by a circumferentially extending recess.

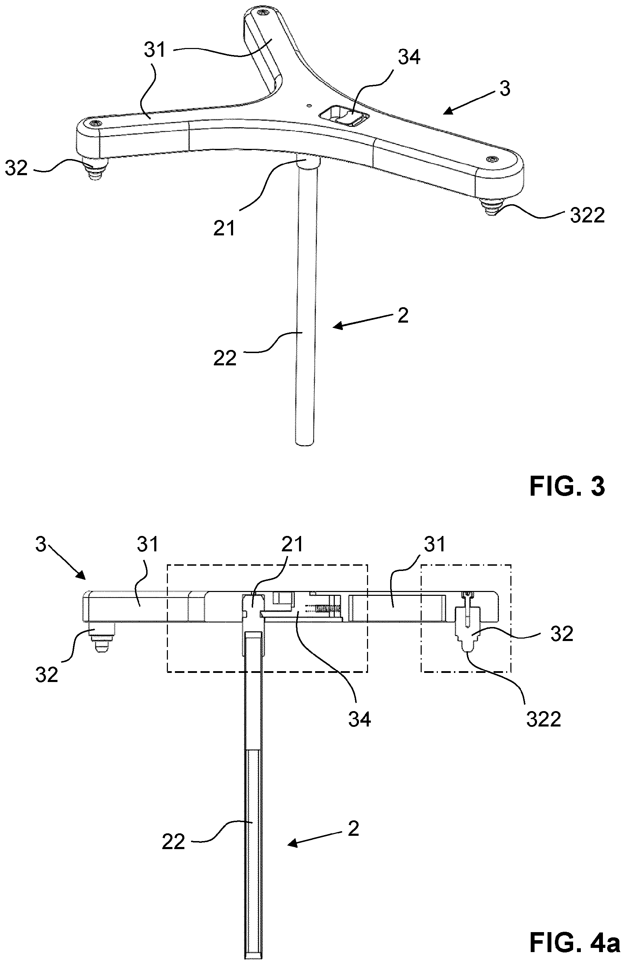

The holding device 3, which is shown in Figure 3, as a whole has the shape of a Y with three radially extending legs 31. The three radially extending legs 31 are joined in a center portion of the holding device 3. Due to the general Y-shape of the holding device 3, the angular distance between a first and a second of the legs 31 is smaller than the angular distance between a third leg 31 and each of the first and the second legs 31, as can be seen from Figure 3. The three legs 31 all extend within the same plane, meaning that the holding device has an overall flat configuration.

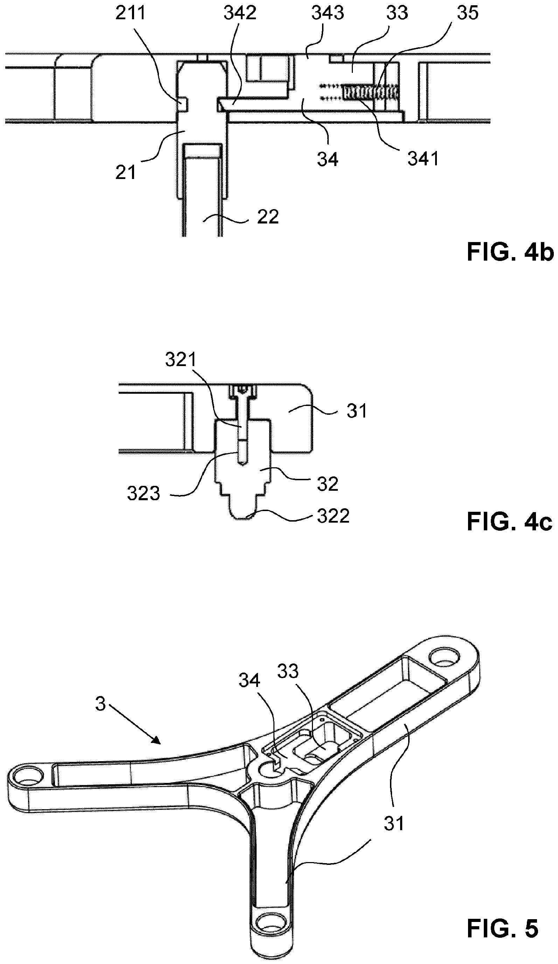

The medical imaging phantom 2 can releasably be attached to the center portion of the holding device 3 via its mounting portion 21. For this purpose, the medical imaging phantom 2 is inserted with its mounting portion 21 into a coupling aperture 33 that is provided at the underside of the center portion of the holding device 3 (see Figures 4a, 4b and 5). The coupling aperture 33 is adapted to the mounting portion 21 as concerns its dimensions. Within the coupling aperture 33, a displaceable latch slider 34 is provided. The latch slider 34 is force-biased by a pressure spring 35 (Figure 4b) in a closing direction with respect to the coupling aperture 33. The latch slider 34 comprises a latch tongue 342 which is pressed by the pressure spring 35 into the recessed latch structure 211, after the mounting portion 21 of the medical imaging phantom 2 has been into the coupling aperture 33. In this way, the medical imaging phantom 2 can be firmly attached to the holding device 3.

For facilitating the insertion of the mounting portion 21 of the medical imaging phantom 2 into the coupling aperture 33 and for removing the medical imaging phantom from the holding device 3, the latch slider 34 comprises an actuation portion 343, which is accessible from the upper side of the holding device 3, i.e. from the opposite side of the holding device 3, compared to the side in which the medical imaging phantom 2 is inserted into the holding device 3. Using the actuation portion 343, the latch slider 34 can be displaced against the biasing force of the pressure spring 35, in order to remove the medical imaging phantom 2 from the holding device 3 or to facilitate the insertion of the mounting portion 21 into the coupling aperture 33. The accessibility of the latch slider 34 from the opposite side of the holding device 3 considerably facilitates the insertion and removal of the medical imaging phantom 2 into and from the holding device 3.

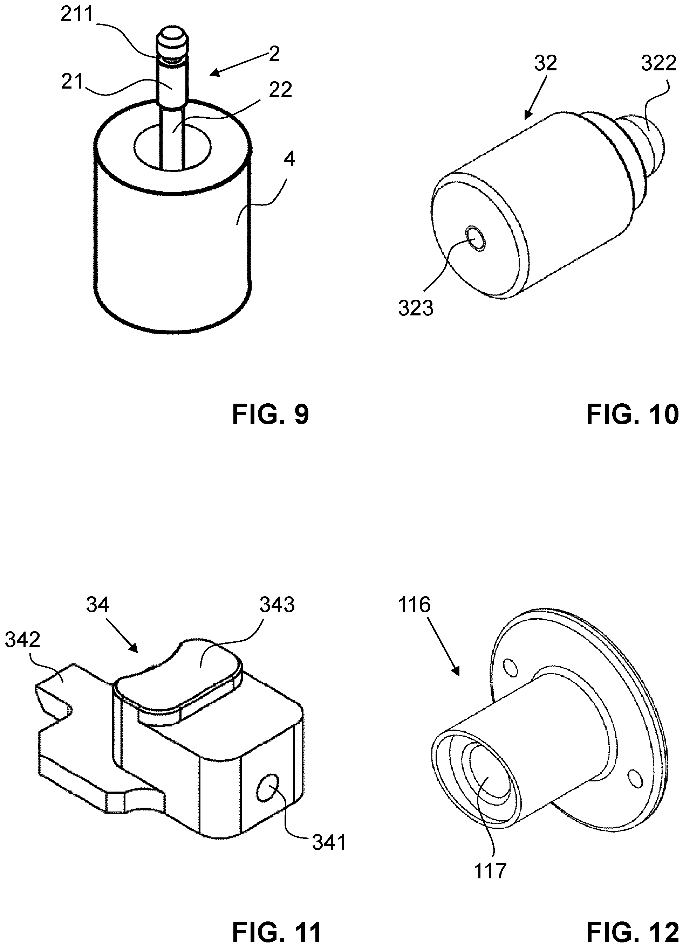

In Figure 11, the latch slider 34 with the latch tongue 342 and the actuation portion 343 is shown individually. As can be seen, the latch slider 34 also comprises a blind hole 341 on the side opposite to the latch tongue 342, which blind hole 341 serves to receive and guide the pressure spring 35.

As can be seen from Figures 3, 4 and 4c, an attachment element 32 is provided in the region of each free end of the legs 31. The attachment element 32 is formed in each case by a protrusion at the underside of the holding device 3. Thus, the attachment elements 32 extend in the same direction and in parallel to the medical imaging phantom 2, when it is attached to the holding device 3 as intended. One of the identically designed attachment elements 32 is shown alone in Figure 10. As can be seen from the Figures, the attachment elements 32 comprise have a generally cylindrical shape with a tipped end that forms a positioning tip 322, which allows a particularly precise positioning of the holding device 3 on the axial end face 113 of the detector ring 11. For fixing the attachment element 32 to a respective leg 31 of the holding device 3 by means of a fixation screw 321, the body of each attachment element 32 comprises a screw bore 323 at the opposite side with regard to the positioning tip 322 (see Figure 4c).

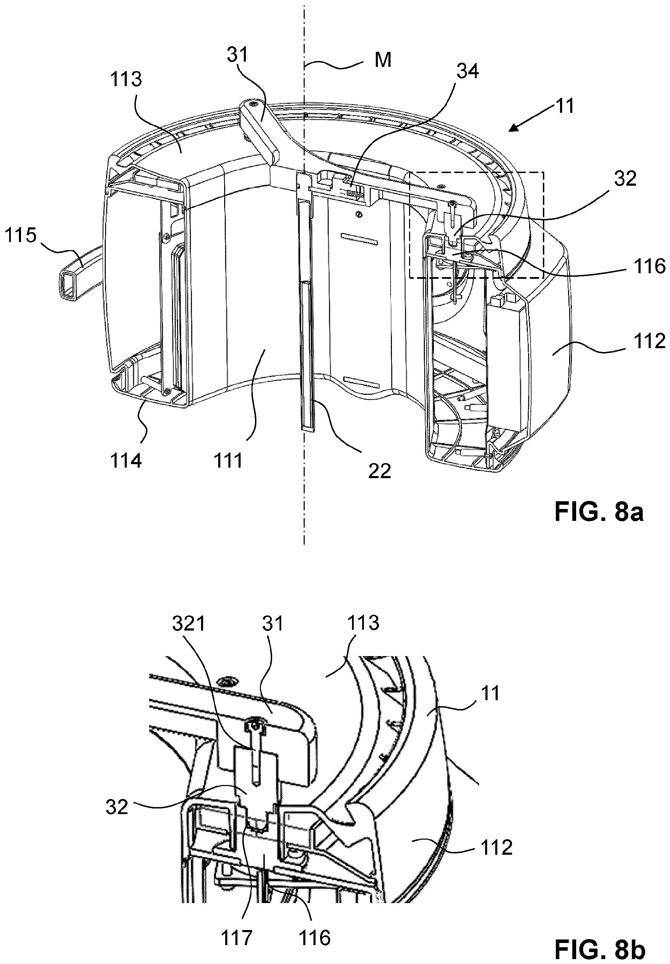

The attachment elements 32 are adapted in each case to engage with their positioning tip 322 a coupling element 116 provided on the axial end face 113 of the detector ring 11. The coupling elements 116, of which one is shown individually in Figure 12, comprise an indentation 117 for receiving the positioning tip 322 of a respective attachment element 32 (see Figure 8b). Due to the complementary design of the indentation 117 and the positioning tip 166, the position of the holding device 3 on the detector ring 11 is precisely defined.

In order to facilitate the attachment and for holding the holding device 3 on the detector ring 11, each of the attachment elements 32 forms a magnet and each of the respective coupling elements 116 is made of a ferromagnetic material, or vice versa, such that the attachment element 32 and the respective coupling element 116 are magnetically attracted. Due to the shapes of the attachment elements 32 and the coupling elements 116 in combination with their magnetic attraction, a particularly easy, but nevertheless precise and accurate positioning of the holding device 3 with the attached medical imaging phantom 2 on the axial end face 113 of the detector ring 11 can be achieved.

As can be seen from Figures 6 to 8a, the holding device 3, when being attached to the detector ring 11 as intended, does not protrude into the main opening of the detector ring 11, but is instead arranged flat on the axial end face 113, i.e. outside of the main opening, of the detector ring. In this mounted state, the main longitudinal axis of the radioactive cylinder portion 22 of the medical imaging phantom 2 coincides with the main longitudinal axis M of the detector ring 11. Along the axial direction, the radioactive cylinder portion 22 is arranged centrally within the detector ring 11.

Figure 9 shows the medical imaging phantom 2 in a state in which it is detached from the holding device 3 and, for storage purposes, inserted in a storage container 4. The storage container 4 forms a nuclear shield with respect to the radioactive material contained in the medical imaging phantom 2, and, therefore, is preferably made of lead. In technical jargon, the storage container 4 is sometimes also referred to as "lead pig".

For the daily routing testing, after coupling the medical imaging phantom 2 with the holding device 3 and attaching the holding device 3 to the axial end face 113 of the detector ring 11, data is acquired based on the radioactive emission from the radioactive cylinder portion 22 arranged in the detector ring 11. For the data acquisition, a standardized sequence is carried out. The obtained data is post-processed, analyzed, optionally displayed, and compared to reference data. Based on certain predefined evaluation criteria, it can be decided by an operator or automatically by e.g. a software, whether the quality standards of the PET-scanning apparatus 1 are met or not. If the required quality standards are not met, a calibration or re-calibration procedure can then be carried out as a next step, which can again be based on the acquisition of data from the medical imaging phantom 2. The data acquisition and post-processing for the daily routine testing and/or the calibration procedure can focus on singles and/or coincidences.

The invention is of course not limited on the preceding presented embodiments and examples, and a plurality of modifications is possible. For example, the holding device does not necessarily need to have the shape of a Y with a plurality of legs. It would for example also be conceivable for the holding device to have as a whole the shape of a flat circular plate. In other embodiments, the holding device could also comprise a first part and, separate therefrom, a second part, wherein the first part is adapted to be attached to the axial end face 113 and the second part is adapted to be attached to the axial end face 114 of the detector ring 11, in order to hold the medical imaging phantom 2 in an even firmer and more stable way on the detector ring 11. Furthermore, the attachment elements of the holding device and/or the coupling elements of the detector ring could also have a different configuration. A different design is also possible with regard to the coupling mechanism between the holding device and the medical imaging phantom. It would also be conceivable, for example, that the holding device is fixedly, i.e. non-releasably, attached to the medical imaging phantom. A plurality of further modifications is possible.

### LIST OF REFERENCE SIGNS

| | | | |
|---|---|---|---|
| 1 | PET-scanning apparatus | 21 | Mounting portion |
| | | 211 | Latch structure |
| 11 | Detector ring | 22 | Radioactive cylinder portion |
| 111 | Inner surface | | |
| 112 | Outer surface | 3 | Holding device |
| 113 | Axial end face | 31 | Leg |
| 114 | Axial end face | 32 | Attachment element |
| 115 | Handle | 321 | Fixation screw |
| 116 | Coupling element | 322 | Positioning tip |
| 117 | Indentation | 323 | Screw bore |
| | | 33 | Coupling aperture |
| 12 | U-shaped portion | 34 | Latch slider |
| 13 | Main supporting structure | 341 | Blind hole |
| 14 | Base structure | 342 | Latch tongue |
| 141 | Wheel | 343 | Actuation portion |
| | | 35 | Pressure spring |
| 15 | Seating support | | |
| 151 | Seat base | 4 | Storage container |
| 152 | Backrest | | |
| 153 | Headrest | R | Axis of rotation |
| 154 | Armrest | D | Displacement direction |
| | | M | Main longitudinal axis |
| 2 | Medical imaging phantom | | |

## Claims

1. A holding device (3) of a medical imaging apparatus, in particular of a medical imaging apparatus in the form of a positron emission tomography (PET)-scanning apparatus (1), for holding a medical imaging phantom (2) in a defined position inside a detector ring (11) of the medical imaging apparatus,
**characterized in that**
the holding device (3) is adapted to be attached to an axial end face (113; 114) of the detector ring (111).

2. The holding device (3) of claim 1, wherein the holding device (3) is adapted to hold the medical imaging phantom (2) in such a way, that the medical imaging phantom (2) extends through the center of the detector ring and in particular along a main longitudinal axis (M) of the detector ring.

3. The holding device (3) of claim 1 or 2, comprising a plurality, in particular three, radially extending legs (31), which are each adapted to be attached to the axial end face (113; 114) of the detector ring (111).

4. The holding device (3) according to one of the preceding claims, having as a whole the shape of a Y.

5. The holding device (3) according to one of the preceding claims, comprising a coupling mechanism, in particular in the form of a latch mechanism (34, 211), for releasably coupling the medical imaging phantom (2) to the holding device (3).

6. The holding device (3) of claim 5, wherein the coupling mechanism (34, 211) can be operated by a user from the opposite side of the holding device (3) with respect to the medical imaging phantom (2), when the medical imaging phantom (2) is attached to the holding device (3).

7. The holding device (3) according to one of the preceding claims, comprising a plurality, in particular three, attachment elements (32) for attaching the holding device (3) to the detector ring (11) in an exact, pre-defined position.

8. The holding device (3) of claim 7, wherein each of the attachment elements (32) forms a protrusion or indentation being adapted to engage a respective complementary indentation (117) or protrusion provided on the axial face (113; 114) of the detector ring (11).

9. The holding device (3) of claims 7 or 8, wherein each of the attachment elements (32) forms a magnet or is made of a ferromagnetic material, in order to be magnetically attracted by a respective ferromagnetic element or a magnet provided on the axial end face (113; 114) of the detector ring (11).

10. A phantom unit comprising the holding device (3) according to one of the preceding claims and a medical imaging phantom (2), which is adapted to be held by the holding device (3).

11. The phantom unit of claim 10, wherein the medical imaging phantom (2) as a whole has the shape of a rod.

12. The phantom unit of claim 11, wherein the phantom unit is adapted to ensure a quality standard of a PET-scanning apparatus (1) and/or to calibrate a PET-scanning apparatus (1), and wherein the medical imaging phantom (2) comprises, for this purpose, an elongated cylinder source formed of a radioactive material, the radioactive material being preferably made from the radionuclide ²²Na or ⁶⁸Ge.

13. A method for ensuring a quality standard of a medical imaging apparatus (1) and/or for calibrating a medical imaging apparatus (1), comprising the step of attaching a holding device (3), which is preferably a holding device (3) according to one of the preceding claims, to an axial end face (113; 114) of a detector ring (11) of the medical imaging apparatus (1) in such a way, that a medical imaging phantom (2) is held in a defined position inside the detector ring (11) by the holding device (3).
